# FASCICULE DE BREVET EUROPEEN

(11) **EP 0 852 944 B1**
(45) Date de publication et mention de la délivrance du brevet: **07.03.2001**
(21) Numéro de dépôt: 97402975.3
(22) Date de dépôt: 09.12.1997
(51) Int. Cl.: A61K 7/48, A61K 7/02

(54) **Utilisation de certains esters de l'acide citrique pour améliorer la rinçabilité des compositions cosmétiques huileuses.**
Verwendung von Citronensäureestern zur Verbesserung der Auswaschbarkeit ölhaltiger kosmetischer Zusammensetzungen
Use of particular esters of citric acid to improve the rinse of oily cosmetic compositions

(30) Priorité: 10.01.1997 FR 9700213
(43) Date de publication de la demande: 15.07.1998
(73) Titulaire: L'OREAL, 75008 Paris (FR)
(72) Inventeur: Gagnebien, Didier, Westfield, 07090, New-Jersey (US); Felardos, Christian, Pav. fond de cour, 94550 Chevilly Larue (FR)
(74) Mandataire: Tezier Herman, Béatrice

(56) Documents cités:
- EP-A- 0 199 131
- EP-A- 0 282 289
- EP-A- 0 521 647
- FR-A- 2 623 422
- US-A- 5 089 531
- US-A- 5 312 968

## Description

L'invention a pour objet l'utilisation de certains esters de l'acide citrique pour améliorer la rinçabilité des compositions cosmétiques huileuses.

Plus particulièrement, l'invention concerne l'amélioration de la rinçabilité de compositions de nettoyage et/ou de démaquillage.

Le nettoyage de la peau est très important pour le soin du visage. Il doit être le plus performant possible car les résidus gras tels que l'excès de sébum, les restes des produits cosmétiques utilisés quotidiennement et les produits de maquillage, notamment les produits "waterproof", résistants à l'eau, s'accumulent dans les replis cutanés et à la surface de la peau et peuvent obstruer les pores de la peau, entraîner l'apparition de boutons. Une mauvaise qualité de nettoyage, et en particulier un mauvais rinçage, sont souvent responsables parmi d'autres facteurs de cause, d'un teint brouillé.

Dans le domaine du soin, les actifs cosmétiques sont parfois utilisés dans des compositions destinées à être rincées, comme par exemple des masques. Après application, de tels produits sont parfois difficiles à éliminer.

Dans le domaine du nettoyage de la peau, le démaquillage des produits "waterproof", résistants à l'eau, des produits sans transfert et des maquillages lourds, comme les maquillages de théâtre, pour être efficace, nécessite l'utilisation de composés huileux.

Les produits, huiles et gels anhydres, crèmes et laits, quoique efficaces, ne sont pas dépourvus d'inconvénients.

Les huiles et les gels anhydres rinçables ont une action nettoyant grâce aux huiles contenues dans ces formulations. Ces huiles permettent la solubilisation des résidus gras et la dispersion des pigments de maquillage. Ces produits sont efficaces et bien tolérés. Ils présentent l'inconvénient d'être lourds, de ne pas mousser et de ne pas conférer de sensation de fraîcheur à l'application, ce qui est pénalisant d'un point de vue cosmétique.
Les crèmes et les laits démaquillants contiennent à la fois des huiles, des émulsionnants et des tensio-actifs détergents en quantité suffisamment faible pour ne pas déstabiliser l'émulsion. Ces produits présentent une rinçabilité insuffisante qui nécessite l'emploi d'une lotion tonique détergente complémentaire pour parfaire le rinçage et l'élimination des salissures. Outre son caractère astreignant, l'utilisation de ce deuxième produit peut entraîner à long terme un assèchement de la peau.

On a cherché à concevoir des produits de soin, de nettoyage et/ou de démaquillage, huileux, présentant une rinçabilité améliorée et une parfaite innocuité.

On connaît, par exemple par le document EP-A-199131, l'utilisation d'esters partiels de l'acide citrique oxyéthylénés en particulier leurs propriétés détergentes, leur pouvoir moussant et leur biodégradabilité. Toutefois, les compositions de l'art antérieur comprenant ces esters citriques sont des compositions aqueuses tensioactives dépourvues d'huiles et comprenant de forts taux de ces esters citriques. L'utilisation de ces esters citriques pour améliorer la rinçabilité des compositions cosmétiques huileuses n'y est ni mentionnée ni suggérée.

Par ailleurs, le document EP-A-282289 décrit l'incorporation d'esters d'acide citrique comme agents adoucissants dans les compositions cosmétiques. Toutefois, les compositions décrites ne sont pas des compositions nettoyantes rinçables.

L'invention a pour but de proposer des compositions cosmétiques qui soient aisément rinçables bien qu'elles comprennent une phase huileuse.

Une composition cosmétique rinçable comprenant :
(i) au moins une phase huileuse ;
(ii) au moins un ester d'acide citrique répondant à la formule (I) : 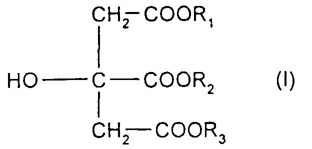 dans laquelle R₁, R₂, R₃, identiques ou différents, représentent un atome d'hydrogène, un métal alcalin ou alcalino-terreux, un cation ammonium ou un cation d'une base organique ou un groupe de formule (II) : -(CₙH₂ₙ-O)ₘ-R₄
   avec
   n = 2, 3, 4
   m = 5 - 30
   et R₄ représente un groupement alkyle en C₈-C₂₄, linéaire ou ramifié, saturé, au moins l'un de R₁, R₂, R₃ étant un groupe de formule (II).

La préparation de tels produits est bien connue de l'homme du métier. On peut par exemple, se référer au document EP-A-199131 où des synthèses de composés répondant à la formule (I) sont illustrées.

Le groupe de formule (II) comprend au moins 5 groupes (CₙH₂ₙ-O) et de préférence au moins 7 (CₙH₂ₙ-O).

Préférentiellement, selon l'invention on choisit des composés répondant à la formule (I) dans laquelle :
n = 2 ou 3,
m = 7 à 20, encore mieux de 7 à 12, et/ou
R₄ représente un groupement alkyle en C₁₂-C₁₈.

Il peut s'agir aussi de mélanges de composés de formule (I).

De préférence, les compositions selon l'invention sont sous la forme d'une lotion huileuse ou d'un gel huileux ou d'une émulsion huile-dans-eau.

Les avantages liés à l'utilisation d'esters citriques selon la formule (I) sont particulièrement remarquables dans des compositions comprenant un fort taux d'huile, c'est-à-dire au moins 40 % en poids d'huile par rapport au poids total de la composition et avantageusement au moins 50 % d'huile, ces compositions étant habituellement difficiles à rincer. Cet effet est particulièrement remarquable dans des compositions comprenant peu de tensioactifs, autres que l'ester citrique, c'est-à-dire moins de 5% en poids par rapport au poids total de la composition, préférentiellement moins de 1% et encore plus préférentiellement pas du tout de tensioactifs, associé à un fort taux d'huiles.

Grâce à ces esters citriques, le démaquillage des produits de maquillage "waterproof' et des produits de maquillage dits « sans transfert » est rendu plus confortable dans la mesure où l'on peut :
. utiliser des compositions huileuses comprenant moins de tensioactifs,
. rincer faiblement la composition huileuse,
. ne pas avoir d'impression de lourdeur sur la peau après le rinçage.

Egalement, le nettoyage et/ou le démaquillage des peaux sensibles est rendu plus confortable, en raison du faible taux ou de l'absence de tensioactifs dans les compositions selon l'invention et de la grande tolérance des esters citriques par la peau.

L'amélioration de la rinçabilité des compositions huileuses par les esters citriques répondant à la formule (I) est d'autant plus remarquable qu'aucun effet analogue n'a été remarqué avec les esters d'acides phosphoriques et d'alcools gras oxyéthylénés.

Les esters d'acides gras et de sorbitane oxyéthylénés n'améliorent la rinçabilité des compositions les comprenant qu'à des taux supérieurs à 10 % en poids par rapport au poids total de la composition huileuse. En outre, les esters de sorbitane oxyéthylénés ayant la plus grande efficacité dans cette application sont les esters oléiques de sorbitane oxyéthylénés, qui présentent l'inconvénient d'être très oxydables. Leur emploi nécessite par conséquent l'utilisation conjointe de forts taux d'anti-oxydants, ce qui n'est pas souhaitable du point de vue de la tolérance de ces compositions par la peau.

En revanche, les esters citriques selon la formule (I) présentent un tel effet à partir de concentrations de 0,1 % en poids par rapport au poids total de la composition et ne sont pas oxydables.

De préférence, ils sont introduits dans les compositions selon l'invention à des concentrations allant de 0,5 à 10 % en poids par rapport au poids total de la composition, et encore plus préférentiellement de 0,5 à 5 %.

Les constituants de la phase huileuse peuvent être choisis, seuls ou en mélanges, parmi les différents corps gras, les huiles d'origine végétale, animale ou minérale, les cires naturelles ou synthétiques, et analogues.

On peut citer parmi les huiles utilisables dans la présente invention les huiles d'origine végétale ou animale, comme par exemple le perhydrosqualène, le squalane, l'huile de coprah, l'huile de macadamia, l'huile de vison, l'huile de tortue, l'huile de soja, l'huile de pépins de raisin, l'huile de sésame, l'huile de maïs, l'huile de colza, l'huile de tournesol, l'huile de coton, l'huile d'avocat, l'huile d'olive, l'huile de ricin, l'huile de jojoba, l'huile d'arachide ; des huiles d'hydrocarbures telles que des huiles de paraffine, la vaseline ; des huiles de silicone comme les polyméthylsiloxanes, les polyméthylphénylsiloxanes, des polysiloxanes modifiés par des acides gras, des polysiloxanes modifiés par des alcools gras, des polysiloxanes modifiés par des polyoxyalkylènes, des silicones fluorées ; des huiles perfluorées et/ou organofluorées ; des acides gras supérieurs tels que l'acide myristique, l'acide palmitique, l'acide stéarique, l'acide béhénique, l'acide oléique, l'acide linoléique, l'acide linolénique, l'acide isostéarique, des alcools gras supérieurs tels que le cétanol, l'alcool stéarylique, l'alcool oléique : des mono et des di-esters parmi lesquels on peut citer en particulier le myristate d'isopropyle, le palmitate d'isopropyle, le stéarate de butyle, le laurate d'hexyle, l'isononanoate d'isononyle, le palmitate de 2-éthyl hexyle, le laurate de 2-hexyl décyle, le palmitate de 2-octyl décyle, le myristate de 2-octyl dodécyle, le succinate de 2-diéthylhexyle, le malate de diisostéaryle, le lactate de 2-octyl dodécyle, le triisostéarate de glycérine, le triisostéarate de glycérine, l'adipate de di-n-butyle, l'adipate de di-(2-éthyl hexyle), le dioléate d'éthylène glycol, le di-isotridécanoate d'éthylène glycol, le di-isostéarate d'éthylène glycol, le di-caprylate de néopentylglycol.

Bien entendu, la phase grasse peut également contenir un ou plusieurs adjuvants cosmétiques lipophiles classiques.

De préférence, on choisit d'introduire au moins une huile polaire dans la phase grasse, cette huile contribuant à la solubilisation de l'ester citrique dans la composition huileuse. Parmi les huiles polaires utilisables dans la présente invention, on peut citer : les triglycérides, les esters de l'acide benzoïque, les esters gras de polyol.

Avantageusement, cette huile polaire représente au moins 25 % en poids par rapport au poids total de la phase grasse.

Les compositions selon l'invention peuvent également comprendre tous les ingrédients habituels bien connus de l'homme du métier tels que les conservateurs, les gélifiants, les épaississants, les antioxydants, les parfums, les filtres, les matières colorantes, les actifs hydrophiles ou lipophiles.

Avantageusement, l'invention concerne des compositions pour le nettoyage et/ou le démaquillage de la peau.

L'invention a pour objet l'utilisation d'esters citriques selon la formule (I) pour améliorer la rinçabilité des compositions cosmétiques huileuses.

Cette utilisation comporte essentiellement deux modes de réalisation préférés :
- dans des compositions huileuses selon l'invention telles que décrites ci-dessus, ou
- dans des lotions aqueuses de rinçage ou lotions toniques associées à une composition cosmétique huileuse.

Selon ce second mode de réalisation l'ester citrique répondant à la formule (I) est de préférence introduit en quantités allant de 0,1 % à 10 % en poids par rapport au poids total de la lotion, et préférentiellement de 0,5 à 5 %. En outre, une telle lotion peut comprendre tous les ingrédients habituels bien connus de l'homme du métier tels que les conservateurs, les antioxydants, les parfums, les filtres, les matières colorantes, les actifs hydrophiles ou lipophiles.

Avantageusement, on introduit moins de 5%, avantageusement moins de 1%, et préférentiellement pas du tout de tensioactifs en plus de l'ester citrique selon (I) dans cette lotion, ce qui permet d'avoir une bonne rinçabilité sans les désavantages liés aux tensio-actifs habituels, dont l'irritation et l'assèchement de la peau.

Les compositions cosmétiques huileuses destinées à être rincées par de telles lotions répondent avantageusement aux caractéristiques décrites ci-dessus pour les compositions huileuses (gels huileux, lotions huileuses ou émulsions huile-dans-eau) selon l'invention, sans toutefois que ces compositions huileuses contiennent nécessairement des esters citriques.

Selon ce second mode de réalisation, l'utilisateur se nettoie et/ou se démaquille à l'aide de la composition huileuse puis dans un second temps il parfait le rinçage de la peau à l'aide de la lotion aux esters citriques.

### EXEMPLES

Dans les exemples tous les pourcentages sont donnés en poids de matière active par rapport au poids total de la composition.

### Exemple 1 : huile démaquillante

- Huile de ricin 29 %
- Palmitate d'éthyl-2 hexyle 70 %
- Monoester citrique d'alcool de coco oxyéthyléné (9 OE) 1 %

### Exemple 2 : démaquillant lèvres

- Huile de ricin 40 %
- Palmitate d'éthyl-2 hexyle 54 %
- Monoester citrique d'alcool de coco oxyéthyléné (7 OE) 1 %
- Alcool éthylique 5 %

### Exemple 3 : Emulsion démaquillante

- Palmitate d'éthyl-2 hexyle 60 %
- Gomme de xanthane 1 %
- Diester citrique d'alcool de coco oxyéthyléné (9 OE) 1 %
- Conservateur 0,5 %
- Eau déminéralisée qsp 100 %

Les compositions des exemples 1 à 3 permettent d'obtenir un démaquillage efficace et très doux de la peau, sans que l'utilisation complémentaire d'une lotion comprenant des tensioactifs soit nécessaire.

## Revendications

1. Utilisation d'au moins un ester d'acide citrique répondant à la formule (I) : 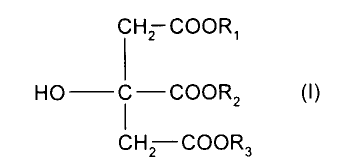 dans laquelle R₁, R₂, R₃, identiques ou différents, représentent un atome d'hydrogène, un métal alcalin ou alcalino-terreux, un cation ammonium ou un cation d'une base organique ou un groupe de formule (II) : -(CₙH₂ₙ-O)ₘ-R₄
avec
n = 2, 3, 4
m = 5 - 30,
et R₄ représente un groupement alkyle en C₈-C₂₄, linéaire ou ramifié, saturé, au moins l'un de R₁, R₂, R₃ étant un groupe de formule (II),
pour améliorer la rinçabilité des compositions cosmétiques huileuses.

2. Utilisation selon la revendication 1, caractérisée en ce que, dans la formule (I) :
n = 2 ou 3,
m = 7 à 20, et/ou
R₄ représente un groupement alkyle en C₁₂-C₁₈.

3. Utilisation selon la revendication précédente, caractérisée en ce que, dans la formule (I), m = 7 à 12.

4. Utilisation selon l'une quelconque des revendications précédentes, caractérisée en ce que la composition est sous la forme d'une lotion huileuse ou d'un gel huileux ou d'une émulsion huile-dans-eau.

5. Utilisation selon l'une quelconque des revendications précédentes, caractérisée en ce que la composition comprend au moins 40 % d'huile, en poids par rapport au poids total de la composition.

6. Utilisation selon l'une quelconque des revendications précédentes, caractérisée en ce que la composition comprend moins de 5%, et préférentiellement moins de 1% de tensioactifs autres que l'ester citrique, en poids par rapport au poids total de la composition.

7. Utilisation selon l'une quelconque des revendications précédentes, caractérisée en ce que la composition comprend au moins 0,1 % en poids par rapport au poids total de la composition d'au moins un ester citrique selon la formule (I).

8. Utilisation selon l'une quelconque des revendications précédentes, caractérisée en ce que la composition comprend de 0,5 à 10 %, et encore plus préférentiellement de 0,5 à 5 %, d'au moins un ester citrique selon la formule (I), en poids par rapport au poids total de la composition.

9. Utilisation selon l'une quelconque des revendications précédentes, caractérisée en ce que la composition comprend au moins une huile polaire dans la phase grasse.

10. Utilisation selon la revendication précédente, caractérisée en ce que la composition comprend au moins 25% en poids par rapport au poids total de la phase grasse d'au moins une huile polaire.

11. Utilisation selon l'une quelconque des revendications précédentes, caractérisée en ce que la composition est sous la forme d'une composition de nettoyage et/ou de démaquillage de la peau.

12. Utilisation selon l'une quelconque des revendications précédentes, caractérisée en ce que la composition est destinée au démaquillage des produits de maquillage "waterproof" et/ou des produits de maquillage dits « sans transfert ».

13. Utilisation selon l'une quelconque des revendications précédentes, caractérisée en ce que la composition est destinée au démaquillage et/ou au nettoyage des peaux sensibles.

## Patentansprüche

1. Verwendung mindestens eines Citronensäureesters der folgenden Formel (I) zur Verbesserung der Abspülbarkeit von öligen kosmetischen Zusammensetzungen: 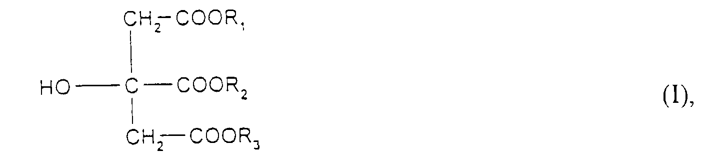 worin die Gruppen R₁, R₂ und R₃, die identisch oder voneinander verschieden sind, ein Wasserstoffatom, ein Alkali- oder Erdalkalimetall, ein Ammoniumkation, ein Kation einer organischen Base oder eine Gruppe der Formel (II): -(CₙH₂ₙ-O)ₘ-R₄ mit n = 2, 3, 4 und m = 5 - 30 bedeuten, wobei die Gruppe R₄ eine geradkettige oder verzweigte, gesättigte C₈₋₂₄-Alkylgruppe bedeutet,
wobei mindestens eine der Gruppen R₁, R₂ oder R₃ eine Gruppe der Formel (II) bedeutet.

2. Verwendung nach Anspruch 1, dadurch gekennzeichnet, daß in Formel (I):
n = 2 oder 3,
m = 7 bis 20, und/oder
R₄ eine C₁₂₋₁₈-Alkylgruppe bedeutet.

3. Verwendung nach dem vorgehenden Anspruch, dadurch gekennzeichnet, daß in Formel (I): m = 7 bis 12.

4. Verwendung nach einem der vorhergehenden Ansprüche, dadurch gekennzeichnet, daß die Zusammensetzung in Form einer öligen Lotion, eines öligen Gels oder einer Öl-in-Wasser-Emulsion vorliegt.

5. Verwendung nach einem der vorhergehenden Ansprüche, dadurch gekennzeichnet, daß die Zusammensetzung mindestens 40 Gew.-% Öl, bezogen auf das Gesamtgewicht der Zusammensetzung, enthält.

6. Verwendung nach einem der vorhergehenden Ansprüche, dadurch gekennzeichnet, daß die Zusammensetzung unter 5 Gew.-% und vorzugsweise weniger als 1 Gew.-% grenzflächenaktive Stoffe. die von den Citronensäureestern verschieden sind, bezogen auf das Gesamtgewicht der Zusammensetzung, enthält.

7. Verwendung nach einem der vorhergehenden Ansprüche, dadurch gekennzeichnet, daß die Zusammensetzung mindestens 0,1 Gew.-% mindestens eines Citronensäureesters der Formel (I), bezogen auf das Gesamtgewicht der Zusammensetzung, enthält.

8. Verwendung nach einem der vorhergehenden Ansprüche, dadurch gekennzeichnet, daß die Zusammensetzung 0,5 bis 10 Gew.-% und noch bevorzugter 0,5 bis 5 Gew.-% mindestens eines Citronensäureesters der Formel (I), bezogen auf das Gesamtgewicht der Zusammensetzung, enthält.

9. Verwendung nach einem der vorhergehenden Ansprüche, dadurch gekennzeichnet, daß die Zusammensetzung in der Fettphase mindestens ein polares Öl enthält.

10. Verwendung nach dem vorhergehenden Anspruch, dadurch gekennzeichnet, daß die Zusammensetzung mindestens 25 Gew.-% mindestens eines polaren Öls, bezogen auf das Gesamtgewicht der Fettphase, enthält.

11. Verwendung nach einem der vorhergehenden Ansprüche, dadurch gekennzeichnet, daß die Zusammensetzung in Form einer Zusammensetzung zur Reinigung und/oder zum Abschminken der Haut vorliegt.

12. Verwendung nach einem der vorhergehenden Ansprüche, dadurch gekennzeichnet, daß die Zusammensetzung zum Abschminken von "Waterproof"-Schminkprodukten und/oder Schminkprodukten "ohne Transfer" bestimmt ist.

13. Verwendung nach einem der vorhergehenden Ansprüche, dadurch gekennzeichnet, daß die Zusammensetzung zum Abschminken und/oder zur Reinigung von empfindlicher Haut bestimmt ist.

## Claims

1. Use of at least one citric acid ester corresponding to the formula (I): 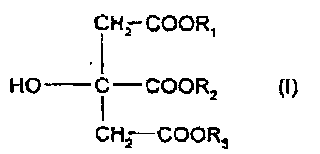 in which R₁, R₂ and R₃, which are identical or different, represent a hydrogen atom, an alkali or alkaline earth metal, an ammonium cation or a cation of an organic base, or a group of formula (II) : - (CₙH₂ₙ-O)ₘ-R₄
with
n = 2, 3 or 4
m = 5-30,
and R₄ representing a saturated, linear or branched, C₈-C₂₄ alkyl group, at least one of R₁, R₂ and R₃ being a group of formula (II),
for improving the rinsability of oily cosmetic compositions.

2. Use according to Claim 1, characterized in that, in the formula (I):
n = 2 or 3,
m = 7 to 20, and/or
R₄ represents a C₁₂-C₁₈ alkyl group.

3. Use according to the preceding claim, characterized in that, in the formula (I), m = 7 to 12.

4. Use according to any one of the preceding claims, characterized in that the composition is in the form of an oily lotion or of an oily gel or of an oil-in-water emulsion.

5. Use according to any one of the preceding claims, characterized in that the composition comprises at least 40% of oil by weight with respect to the total weight of the composition.

6. Use according to any one of the preceding claims, characterized in that the composition comprises less than 5% and preferably less than 1% of surfactants, other than the citric ester, by weight with respect to the total.weight of the composition.

7. Use according to any one of the preceding claims, characterized in that the composition comprises at least 0.1% by weight with respect to the total weight of the composition of at least one citric ester according to the formula (I).

8. Use according to any one of the preceding claims, characterized in that the composition comprises from 0.5 to 10% and more preferably still from 0.5 to 5% of at least one citric ester according to the formula (I) by weight with respect to the total weight of the composition.

9. Use according to any one of the preceding claims, characterized in that the composition comprises at least one polar oil in the fatty phase.

10. Use according to the preceding claim, characterized in that the composition comprises at least 25% by weight with respect to the total weight of the fatty phase of at least one polar oil.

11. Use according to any one of the preceding claims, characterized in that the composition is in the form of a composition for cleansing and/or removing make-up from the skin.

12. Use according to any one of the preceding claims, characterized in that the composition is intended for removing waterproof make-up products and/or so-called "transfer-free" make-up products.

13. Use according to any one of the preceding claims, characterized in that the composition is intended for removing make-up from and/or for cleansing sensitive skin.
